# FASCICULE DE BREVET EUROPEEN

(11) **EP 1 453 969 B1**
(45) Date de publication et mention de la délivrance du brevet: **11.10.2006**
(21) Numéro de dépôt: 02804610.0
(22) Date de dépôt: 12.12.2002
(51) Int. Cl.: C12Q 1/04

(54) **PROCEDE DE DETECTION DE MICROORGANISMES**
VERFAHREN ZUM NACHWEIS VON MIKROORGANISMEN
METHOD FOR DETECTING MICROORGANISMS

(30) Priorité: 13.12.2001 FR 0116106
(43) Date de publication de la demande: 08.09.2004
(73) Titulaire: Rambach, Alain, F-75006 Paris (FR)
(72) Inventeur: Rambach, Alain, F-75006 Paris (FR)
(74) Mandataire: Callon de Lamarck, Jean-Robert
(86) Numéro de dépôt international: PCT/FR2002/004310
(87) Numéro de publication internationale: WO 2003/050289

(56) Documents cités:
- EP-A- 0 103 915
- WO-A-00/53799
- WO-A-95/04156
- WO-A-97/39103
- WO-A-98/48042
- US-A- 5 210 022
- US-A- 6 087 156
- US-A- 6 130 057

## Description

La présente invention se rapporte à une méthode pour détecter des microorganismes présents dans un échantillon, dans laquelle on inocule un milieu de culture avec ledit échantillon, ledit milieu de culture contenant de préférence des agents chromogènes libérant des chromophores en présence des microorganismes cibles ou des agents fluorogènes, ledit milieu de culture étant dans un récipient présentant un fond coloré, la détection étant facilitée par le contraste entre le fond coloré du récipient et les colonies isolées.

La détection rapide de microorganismes présents dans des échantillons biologiques, alimentaires, ou en milieu hospitalier est indispensable, afin de définir le plus rapidement possible les mesures à prendre en cas de contamination.

Il apparaît également important de pouvoir différencier les différents contaminants, afin de pouvoir procéder à des désinfection adaptées.

Des méthodes existent, comme la PCR, qui est rapide et spécifique, mais qui nécessite l'emploi d'appareils comme les thermocycleurs, et qui ne peut pas toujours être mise en oeuvre en milieu hospitalier. Par ailleurs, cette méthode peut demander une préparation des échantillons à tester, ce qui n'est pas toujours aisé à mettre en oeuvre.

Des méthodes basées sur la culture des microorganismes contaminants sur des milieux adaptés, et sur leur identification du fait de l'ajout d'agents chromogènes libérant un chromophore après action d'une enzyme spécifique du microorganisme ont été décrits.

Ainsi, les demandes de brevets WO 00/53799, WO 00/46345, WO 97/39103, WO 95/04156, WO 94/09152 US-A-6 087 156, WO 98/48042 et US-A-6 130 057 décrivent des milieux de culture pour identifier les staphylocoques, les bactéries anaérobies ou les coliformes entérohémorragiques, les salmonelles ou d'autres microorganismes tels que les candida.

Toutefois, les milieux utilisés étant en général relativement transparents, il peut parfois s'avérer difficile de bien déterminer les couleurs des chromophores libérés, du fait d'un manque de contraste suffisant. Ainsi, l'utilisation du milieu C décrit dans la demande de brevet WO 95/04156 permet une bonne discrimination des différentes levures candida, mais les couleurs obtenues sont claires, ce qui peut être problématique pour certains utilisateurs.

De même, la méthode décrite dans la demande WO 00/46345 pour identifier les staphylocoques peut nécessiter d'ajouter un agent de contraste au milieu de culture, par exemple un opacifiant.

Un tel besoin de contraste se fait également sentir pour des milieux pour détecter les salmonelles, tel que le Rambach Agar vendu par la société Chromagar (Paris, France).

On a notamment proposé l'ajout de kaolin pour opacifier les milieux, notamment pour détecter *E. coli,* à l'aide du phénylglucuronide. Toutefois, l'ajout de ce type composant, outre qu'il augmente les coûts de fabrication du milieu, peut s'avérer poser de nombreux problèmes pour la fabrication industrielle de milieux de détection (notamment précipitation).

Le document EP0103915 décrit une méthode pour la détection visuelle d'une intensité colorée d'un liquide dans une série de récipients avec différentes concentrations de colorants, lesdits récipients pouvant être colorés.

La présente invention se propose de répondre au problème posé par le manque de contraste observé entre les colonies isolées et le milieu de culture, en particulier lorsque ces colonies sont colorées du fait de la présence d'agents chromogènes dans le milieu de culture.

Ainsi, la présente invention se rapporte à une méthode pour détecter des microorganismes présents dans un échantillon comprenant les étapes consistant à :
a) préparer un milieu de culture adapté auxdits microorganismes cibles,
b) couler ledit milieu dans un récipient présentant un fond coloré,
c) inoculer le milieu de culture solide de a) avec ledit échantillon ou un inoculum dérivé de l'échantillon et incuber,
d) détecter la présence desdits microorganismes sur ledit milieu de culture en utilisant le contraste entre la couleur du fond du récipient et les colonies isolées.

Dans un mode de réalisation préféré, l'invention se rapporte à une méthode pour détecter des microorganismes présents dans un échantillon par libération enzymatique de chromophores à partir d'agents chromogènes présents dans un milieu de culture adapté auxdits microorganismes comprenant les étapes consistant à:
a) préparer un milieu de culture adéquat contenant les agents chromogènes appropriés à la détection des microorganismes cibles,
b) couler ledit milieu dans un récipient présentant un fond coloré,
c) inoculer le milieu de culture solide de a) avec ledit échantillon ou un inoculum dérivé de l'échantillon, et incuber,
d) détecter la présence des microorganismes présents dans ledit échantillon, sur ledit milieu de culture, en utilisant le contraste lié à la couleur du fond du récipient par rapport à la coloration due aux chromophores libérés.

Dans un autre mode de réalisation, le milieu de culture contient des produits lui donnant une coloration (par exemple, le McConkey Agar...).

Dans un autre mode de réalisation, le milieu de culture contient des agents fluorogènes, libérant des fluorophores en présence des microorganismes cibles. On peut notamment utiliser le 4-méthyl umbelliféryl, en tant que fluorophore, par exemple lié à un galactoside (4-methylumbelliferyl-β-D-galactoside).

Dans un mode de réalisation préféré, le récipient selon l'invention est de forme ronde et notamment une boîte de Pétri, d'un diamètre allant de 40mm à 210mm. De préférence, le diamètre du récipient selon l'invention est égal à 47mm, 55mm, 90mm, 100mm ou 200mm.

Dans un mode de réalisation préféré de l'invention, la surface du récipient est telle qu'il est possible d'inoculer le milieu de culture contenu dans le récipient de telle façon qu'on obtienne croissance de colonies isolées. Ainsi, la surface du récipient selon l'invention est de préférence supérieure à environ 15-17cm².

Dans un mode de réalisation de la présente invention, ledit récipient présente un fond coloré opaque. Dans un autre mode de réalisation, ledit récipient présente un fond coloré transparent. De préférence, ledit récipient présente un fond d'une couleur choisie parmi les couleurs bleue, rouge, jaune, noire ou blanche opaque.

Dans un mode de réalisation de l'invention, lesdits chromophores ou autres produits colorés sont libérés par hydrolyse du fait de l'action d'une enzyme spécifique du microorganisme sur les agents chromogènes présents dans le milieu de culture., et le(s)dit(s) agent(s) chromogène(s) est (sont) choisi(s) parmi les substrats des enzymes liés au métabolisme des sucres, et notamment dans le groupe constitué des substrats de la β-glucosidase, des substrats de la β-galactosidase, et des substrats de la β-glucuronisase, et des substrats de la phosphatase.

De préférence, le(s)dit(s) chromophore(s) est (sont) choisi(s) dans le groupe constitué des dérivés indoxyle, halogéno-indoxyle (bromo-indoxyle, chloro-indoxyle, fluoro-indoxyle, iodo-indoxyle, dichloro-indoxyle, chloro-bromo-indoxyle, tri-chloro-indoxyle), méthyl-indoxyle, et hydroxy-quinoline, en particulier parmi les dérivés suivants : 6-chloro-indoxyle, 5-bromo-indoxyle, 3-bromo-indoxyle, 6- fluoro-indoxyle, 5-iodo-indoxyle, 4,6-dichloro-indoxyle, 6,7-dichloro-indoxyle, 5-bromo-4-chloro-indoxyle, 5-bromo-6-chloro-indoxyle, 4,6,7-trichloro-indoxyle, N-méthyl-indoxyle ou 8-hydroxy-quinoline.

Afin de pouvoir limiter la croissance des microorganismes qui ne sont pas recherchés, il est avantageux que le milieu de culture contienne également des facteurs sélectifs pour les microorganismes recherchés.

Dans un mode de réalisation particulier, le milieu de culture contient également une concentration élevée de saccharose ou du glucose.

La méthode de l'invention est avantageusement mise en oeuvre lorsque le microorganisme cible est de l'espèce Candida, en particulier avec le Chromagar Candida décrit dans WO 95/04156. On utilise alors un récipient coloré, transparent ou opaque, la couleur dépendant de la nature de la levure la plus recherchée.

La méthode de l'invention est également particulièrement adaptée lorsque ledit microorganisme cible est de l'espèce Staphylococcus et l'on choisit alors de préférence ledit récipient afin qu'il présente un fond coloré opaque, de préférence blanc opaque.

On peut aussi mettre la méthode en oeuvre lorsque ledit microorganisme cible est de l'espèce *Vibrio,* de l'espèce des coliformes et en particulier des bactéries *E. coli* entérohémorragiques.

On utilise en particulier les enseignements des demandes de brevets WO 00/53799, WO 00/46345, WO 97/39103, WO 95/04156, WO 94/09152, afin de définir les couleurs des microorganismes en fonction de la nature des milieux obtenus.

L'invention se rapporte également à un kit de détection de microorganismes, constitué de la combinaison d'un récipient présentant un fond coloré avec un milieu de culture contenant de préférence des agents chromogènes libérant des chromophores en présence desdits microorganismes cibles.

L'invention a également pour objet l'utilisation d'un récipient présentant un fond coloré en tant que support dans lequel on coule un milieu de culture contenant de préférence des agents chromogènes libérant des chromophores après action d'enzymes spécifiques présentes dans des souches de microorganismes.

La couleur des récipients utilisés (en général des boîtes de Pétri) est avantageusement choisie de telle sorte qu'elle présente un contraste utile avec les colonies de microorganismes, ou les chromogènes utilisés. Le contraste utile est défini comme un contraste aidant à la discrimination des couleurs des colonies, par rapport au fond de la boîte. Ainsi, lorsque le milieu de culture ne contient pas de chromogènes, les colonies sont généralement claires et le fond du récipient est plutôt foncé.

La couleur du fond de la boîte est aussi choisie en fonction de la couleur des chromophores libérés. Ainsi, le chromogène bleu 5-bromo-4-chloro-3-indoxyl présente une absorption à 615 nm, que l'on peut aisément différentier lorsque l'on utilise des boîtes rouges (environ 515 nm). Pour les chromogènes produisant des colonies mauve, rouge, ou pourpre, on utilise des boîtes avec une couleur telle que la longueur d'onde soit éloignée, par exemple des boîtes jaunes. Lorsque l'on obtient des colonies de couleur claire, on utilise des boîtes de couleur foncée et/ou opaques.

| Chromogène | Couleur | Absorption max (nm) | Couleur de la boîte (exemple) |
|---|---|---|---|
| 5-Bromo-4-chloro-3-indoxyl | bleue | 615 | rouge |
| 5-Bromo-6-chloro-3-indoxyl | rouge-pourpre (magenta) | 565 | jaune |
| 6-chloro-3-indoxyl | gris-pourpre (saumon) | 540 | Blanc opaque, ou couleur foncée |

Les chromophores utilisables pour la mise en oeuvre de la présente invention ont alors la formule générale suivante, et sont obtenus par oxydation d'un agent chromogène lié à un substrat enzymatique. Ces chromophores sont insolubles, ce qui induit la formation d'un précipité coloré. On note que les chromophores peuvent être obtenus après action des glycosidases, mais aussi après action de phosphatases.

Une liste d'agents chromogènes peut être obtenue du brevet US 6,130,057.

Formule de chromophores utilisables dans le cadre de l'invention :

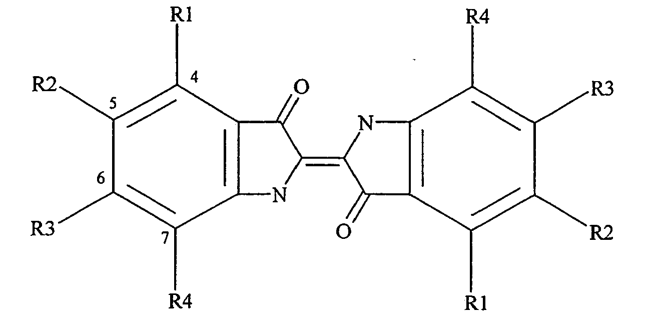

| **R1** | **R2** | **R3** | **R4** | **Couleur** |
|---|---|---|---|---|
| H | H | H | H | Bleu |
| Cl | Br | H | H | Bleu |
| H | Br | Cl | H | Magenta |
| H | H | Cl | H | Saumon |
| H | I | H | H | Pourpre |
| H | Br | H | H | bleu |

Un autre avantage de la méthode et du kit selon l'invention est la possibilité de repérer facilement les boîtes comprenant certains agents. En effet, les milieux contenant des agents chromogènes sont incolore avant réaction des microorganismes et libération des chromophores. Leur conditionnement dans des boîtes de couleur s'avère donc utile pour ranger et retrouver facilement les milieux d'intérêt, la couleur de la boîte étant choisie en fonction des et corrélée aux chromogènes présents dans le milieu, et aux couleurs attendues en présence de microorganismes.

## Revendications

1. Méthode pour détecter des microorganismes présents dans un échantillon comprenant les étapes consistant à :
a) préparer un milieu de culture adapté auxdits microorganismes cibles,
b) couler ledit milieu dans un récipient présentant un fond coloré,
c) inoculer le milieu de culture solide de a) avec ledit échantillon ou un inoculum dérivé de l'échantillon et incuber,
d) détecter la présence desdits microorganismes sur ledit milieu de culture en utilisant le contraste entre la couleur du fond du récipient et les colonies isolées.

2. Méthode selon la revendication 1, **caractérisée en ce que** ledit milieu de culture contient des agents fluorogènes libérant des fluorophores en présence desdits organismes cibles.

3. Méthode selon la revendication 1, **caractérisée en ce que** ledit milieu de culture contient des agents chromogènes appropriés à la détection des microorganismes cibles, par libération enzymatique de chromophores à partir desdits agents chromogènes.

4. Méthode selon l'une des revendications 1 à 3, **caractérisée en ce que** ledit récipient présente un fond coloré opaque.

5. Méthode selon l'une des revendications 1 à 3, **caractérisée en ce que** ledit récipient présente un fond coloré transparent.

6. Méthode selon l'une des revendications 1 à 5, **caractérisée en ce que** ledit récipient présente un fond d'une couleur choisie parmi les couleurs bleue, rouge, jaune, noire, ou blanche opaque.

7. Méthode selon l'une des revendications 3 à 6, **caractérisée en ce que** lesdits chromophores sont libérés par hydrolyse du fait de l'action d'une enzyme spécifique du microorganisme sur les agents chromogènes présents dans le milieu de culture.

8. Méthode selon la revendication 7, **caractérisée en ce que** le(s)dit(s) agent(s) chromogène(s) est (sont) choisi(s) parmi les substrats des enzymes liés au métabolisme des sucres, et notamment dans le groupe constitué des substrats de la β-glucosidase, des substrats de la β-galactosidase, et des substrats de la β-glucuronisase.

9. Méthode selon l'une des revendications 3 à 8, **caractérisée en ce que** le(s)dit(s) chromophore(s) est (sont) choisi(s) dans le groupe constitué des dérivés indoxyle, halogéno-indoxyle (bromo-indoxyle, chloro-indoxyle, fluoro-indoxyle, iodo-indoxyle, dichloro-indoxyle, chloro-bromo- indoxyle, tri-chloro-indoxyle), méthyl-indoxyle, et hydroxy-quinoline, en particulier parmi les dérivés suivants : 6-chloro-indoxyle, 5-bromo-indoxyle, 3-bromo-indoxyle, 6-fluoro-indoxyle, 5-iodo-indoxyle, 4,6-dichloro-indoxyle, 6,7-dichloro-indoxyle, 5-bromo-4-chloro-indoxyle, 5-bromo-6-chloro-indoxyle, 4,6,7-trichloro-indoxyle, N-méthyl-indoxyle ou 8-hydroxy-quinoline.

10. Méthode selon l'une des revendications 1 à 9, **caractérisée en ce que** le milieu de culture contient également des facteurs sélectifs pour les microorganismes recherchés.

11. Méthode selon l'une des revendications 1 à 10, **caractérisée en ce que** le microorganisme cible est de l'espèce *Candida.*

12. Méthode selon l'une des revendications 1 à 4 et 7 à 10, **caractérisée en ce que** ledit microorganisme cible est de l'espèce *Staphylococcus* et que ledit récipient présente un fond coloré opaque, de préférence blanc opaque.

13. Méthode selon l'une des revendications 1 à 10, **caractérisée en ce que** ledit microorganisme cible est de l'espèce *Vibrio.*

14. Méthode selon l'une des revendications 1 à 10, **caractérisé en ce que** ledit microorganisme cible est de l'espèce des coliformes et en particulier des bactéries *E. coli* entérohémorragiques.

15. Kit pour la détection de microorganismes, constitué de la combinaison d'un récipient présentant un fond coloré et d'un milieu de culture, la couleur dudit récipient étant choisie de telle sorte qu'elle présente un contraste utile avec la couleur des colonies isolées.

16. Kit selon la revendication 15, **caractérisé en ce que** ledit milieu de culture contient des agents fluorogènes libérant des fluorophores en présence desdits microorganismes cibles.

17. Kit selon la revendication 15, **caractérisé en ce que** ledit milieu de culture contient des agents chromogènes libérant des chromophores en présence desdits microorganismes cibles, la couleur dudit récipient étant choisie de telle sorte qu'elle présente un contraste utile avec la couleur des chromophores libérés.

18. Utilisation d'un récipient présentant un fond coloré en tant que support dans lequel on coule un milieu de culture apte à la croissance de microorganismes, la couleur du fond du récipient permettant un contraste avec les colonies desdits microorganismes.

19. Utilisation selon la revendication 18, **caractérisée en ce que** ledit milieu de culture contient des agents fluorogènes libérant des fluorophores en présence desdits microorganismes cibles.

20. Utilisation selon la revendication 18, **caractérisée en ce que** ledit milieu de culture contient des agents chromogènes libérant des chromophores après action d'enzymes spécifiques présentes dans des souches de microorganismes, la couleur dudit récipient étant choisie de telle sorte qu'elle présente un contraste utile avec la couleur des chromophores libérés.

## Claims

1. Method for detecting microorganisms present in a sample comprising the steps consisting in:
a) preparing a culture medium suitable for said target microorganisms,
b) pouring said medium into a container having a coloured bottom,
c) inoculating the solid culture medium from a) with said sample or an inoculum derived from the sample and incubating,
d) detecting the presence of said microorganisms on said culture medium using the contrast between the colour of the bottom of the container and the colonies isolated.

2. Method according to Claim 1, **characterized in that** said culture medium contains fluorogenic agents releasing fluorophores in the presence of said target organisms.

3. Method according to Claim 1, **characterized in that** said culture medium contains chromogenic agents appropriate for the detection of target microorganisms, by the enzymatic release of chromophores from said chromogenic agents.

4. Method according to one of Claims 1 to 3, **characterized in that** said container has an opaque coloured bottom.

5. Method according to one of Claims 1 to 3, **characterized in that** said container has a transparent coloured bottom.

6. Method according to one of Claims 1 to 5, **characterized in that** said container has a bottom having a colour chosen from the colours blue, red, yellow, black or opaque white.

7. Method according to one of Claims 3 to 6, **characterized in that** said chromophores are released by hydrolysis by virtue of the action of an enzyme specific to the microorganism on the chromogenic agents present in the culture medium.

8. Method according to Claim 7, **characterized in that** said chromogenic agent(s) is (are) chosen from enzyme substrates linked to the metabolism of sugars, and in particular from the group consisting of β-glucosidase substrates, β-galactosidase substrates and β-glucuronisase substrates.

9. Method according to one of Claims 3 to 8, **characterized in that** the said chromophore(s) is (are) chosen from the group consisting of indoxyl, haloindoxyl (bromoindoxyl, chloroindoxyl, fluoroindoxyl, iodoindoxyl, dichoroindoxyl, chlorobromoindoxyl, trichloroindoxyl), methylindoxyl and hydroxyquinoline derivatives, in particular from the following derivatives: 6-chloroindoxyl, 5-bromoindoxyl, 3-bromoindoxyl, 6-fluoroindoxyl, 5-iodoindoxyl, 4,6-dichloroindoxyl, 6,7-dichloroindoxyl, 5-bromo-4-chloroindoxyl, 5-bromo-6-chloroindoxyl, 4,6,7-trichloroindoxyl, N-methylindoxyl or 8-hydroxy-quinoline.

10. Method according to one of Claims 1 to 9, **characterized in that** the culture medium also contains factors selective for the microorganisms being searched for.

11. Method according to one of Claims 1 to 10, **characterized in that** target microorganism is of the *Candida* species.

12. Method according to one of Claims 1 to 4 and 7 to 10, **characterized in that** said target microorganism is of the *Staphylococcus* species and said container has an opaque, preferably an opaque white, coloured bottom.

13. Method according to one of Claims 1 to 10, **characterized in that** said target microorganism is of the *Vibrio* species.

14. Method according to one of Claims 1 to 10, **characterized in that** the said target microorganism is of the species comprising coliforms and in particular enterohemorrhagic bacteria *E. coli.*

15. Kit for detecting microorganisms, consisting of the combination of a container having a coloured bottom and of a culture medium, the colour of said container being chosen such that it exhibits a useful contrast with the colour of the colonies isolated.

16. Kit according to Claim 15, **characterized in that** said culture medium contains fluorogenic agents releasing fluorophores in the presence of said target microorganisms.

17. Kit according to Claim 15, **characterized in that** said culture medium contains chromogenic agents releasing chromophores in the presence of said target microorganisms, the colour of said container being chosen such that it exhibits a useful contrast with the colour of the chromophores released.

18. Use of a container having a coloured bottom as a support into which is poured a culture medium suitable for the growth of microorganisms, the colour of the bottom of the container allowing a contrast with the colonies of said microorganisms.

19. Use according to Claim 18, **characterized in that** said culture medium contains fluorogenic agents releasing fluorophores in the presence of said target microorganisms.

20. Use according to Claim 18, **characterized in that** said culture medium contains chromogenic agents releasing chromophores after the action of specific enzymes present in strains of microorganisms, the colour of said container being chosen such that it exhibits a useful contrast with the colour of the chromophores released.

## Patentansprüche

1. Verfahren zum Nachweisen von Mikroorganismen, die in einer Probe vorhanden sind, umfassend die Schritte, die bestehen aus:
a) Herstellen eines Kulturmediums, das an die Ziel-Mikroorganismen angepasst ist,
b) Gießen des Mediums in einen Behälter, der einen gefärbten Boden aufweist,
c) Impfen des festen Kulturmediums von a) mit der Probe oder einem Inokulum, das von der Probe abstammt, und Inkubieren,
d) Nachweisen der Anwesenheit der Mikroorganismen über dem Kulturmedium, indem der Kontrast zwischen der Farbe des Bodens des Behälters und der isolierten Kolonien verwendet wird.

2. Verfahren nach Anspruch 1, **dadurch gekennzeichnet, dass** das Kulturmedium fluorogene Mittel enthält, welche Fluorophore in Anwesenheit der Ziel-Organismen freisetzen.

3. Verfahren nach Anspruch 1, **dadurch gekennzeichnet, dass** das Kulturmedium chromogene Mittel enthält, die für den Nachweis der Ziel-Mikroorganismen durch enzymatische Freisetzung von Chromophoren aus den chromogenen Mitteln geeignet sind.

4. Verfahren nach einem der Ansprüche 1 bis 3, **dadurch gekennzeichnet, dass** der Behälter einen opaken gefärbten Boden aufweist.

5. Verfahren nach einem der Ansprüche 1 bis 3, **dadurch gekennzeichnet, dass** der Behälter einen transparenten gefärbten Boden aufweist.

6. Verfahren nach einem der Ansprüche 1 bis 5, **dadurch gekennzeichnet, dass** der Behälter einen Boden mit einer Farbe aufweist, die aus den Farben Blau, Rot, Gelb, Schwarz oder opakes Weiß ausgewählt ist.

7. Verfahren nach einem der Ansprüche 3 bis 6, **dadurch gekennzeichnet, dass** die Chromophore durch Hydrolyse aufgrund der Einwirkung eines für den Mikroorganismus spezifischen Enzyms auf die chromogenen Mittel freigesetzt werden, welche in dem Kulturmedium vorliegen.

8. Verfahren nach Anspruch 7, **dadurch gekennzeichnet, dass** das oder die chromogene(n) Mittel aus den Substraten von Enzymen, die mit dem Metabolismus von Zuckern assoziiert sind, und insbesondere aus der Gruppe bestehend aus Substraten von β-Glucosidase, Substraten von β-Galactosidase und Substraten von β-Glucuronidase ausgewählt ist (sind).

9. Verfahren nach einem der Ansprüche 3 bis 8, **dadurch gekennzeichnet, dass** der oder die Chromophor(e) ausgewählt ist (sind) aus der Gruppe bestehend aus Indoxyl-, Halogenindoxyl- (Bromindoxyl-, Chlorindoxyl-, Fluorindoxyl-, lodindoxyl-, Dichlorindoxyl-, Chlorbromindoxyl-, Trichlorindoxyl-), Methylindoxyl- und Hydroxychinolin-Derivaten, insbesondere aus den folgenden Derivaten: 6-Chlorindoxyl, 5-Bromindoxyl, 3-Bromindoxyl, 6-Fluorindoxyl, 5-lodindoxyl, 4,6-Dichlorindoxyl, 6,7-Dichlorindoxyl, 5-Brom-4-chlorindoxyl, 5-Brom-6-chlorindoxyl, 4,6,7-Trichlorindoxyl, N-Methylindoxyl oder 8-Hydroxychinolin.

10. Verfahren nach einem der Ansprüche 1 bis 9, **dadurch gekennzeichnet, dass** das Kulturmedium auch Faktoren enthält, die für die gesuchten Mikroorganismen selektiv sind.

11. Verfahren nach einem der Ansprüche 1 bis 10, **dadurch gekennzeichnet, dass** der Ziel-Mikroorganismus die Art *Candida* ist.

12. Verfahren nach einem der Ansprüche 1 bis 4 und 7 bis 10, **dadurch gekennzeichnet, dass** der Ziel-Mikroorganismus die Art *Staphylococcus* ist und dass der Behälter einen gefärbten opaken, bevorzugt weißen opaken Boden aufweist.

13. Verfahren nach einem der Ansprüche 1 bis 10, **dadurch gekennzeichnet, dass** der Ziel-Mikroorganismus die Art *Vibrio* ist.

14. Verfahren nach einem der Ansprüche 1 bis 10, **dadurch gekennzeichnet, dass** der Ziel-Mikroorganismus aus der Art der Coliforme und insbesondere der enterohämorragischen *E. coli*-Bakterien ist.

15. Kit zum Nachweis von Mikroorganismen, bestehend aus der Kombination eines Behälters, der einen gefärbten Boden aufweist, und eines Kulturmediums, wobei die Farbe des Behälters so ausgewählt ist, dass sie einen nützlichen Kontrast mit der Farbe von isolierten Kolonien bereitstellt.

16. Kit nach Anspruch 15, **dadurch gekennzeichnet, dass** das Kulturmedium fluorogene Mittel enthält, welche in Anwesenheit der Ziel-Mikroorganismen Fluorophore freisetzen.

17. Kit nach Anspruch 15, **dadurch gekennzeichnet, dass** das Kulturmedium chromogene Mittel enthält, welche Chromophore in Anwesenheit der Ziel-Mikroorganismen freisetzen, wobei die Farbe des Behälters so gewählt ist, dass sie einen nützlichen Kontrast mit der Farbe der freigesetzten Chromophore bereitstellt.

18. Verwendung eines Behälters, der einen gefärbten Boden aufweist, als Träger, in den man ein Kulturmedium gießt, das zum Züchten von Mikroorganismen geeignet ist, wobei die Farbe des Bodens des Behälters einen Kontrast mit den Kolonien der Mikroorganismen gestattet.

19. Verwendung nach Anspruch 18, **dadurch gekennzeichnet, dass** das Kulturmedium fluorogene Mittel enthält, die in Anwesenheit der Ziel-Mikroorganismen Fluorophore freisetzen.

20. Verwendung nach Anspruch 18, **dadurch gekennzeichnet, dass** das Kulturmedium chromogene Mittel enthält, die nach Einwirkung von spezifischen Enzymen, die in Stämmen von Mikroorganismen vorhanden sind, Chromophore freisetzen, wobei die Farbe des Behälters so gewählt ist, dass sie einen nützlichen Kontrast mit der Farbe der freigesetzten Chromophore bereitstellt.
